# EUROPEAN PATENT SPECIFICATION

(11) **EP 0 704 202 B1**
(45) Date of publication and mention of the grant of the patent: **05.04.2000**
(21) Application number: 95113029.3
(22) Date of filing: 18.08.1995
(51) Int. Cl.: A61K 7/16

(54) **Anti-cariogenic agent**
Antikariogenisches Mittel
Composé anti-cariogène

(30) Priority: 18.08.1994 JP 19426494
(43) Date of publication of application: 03.04.1996
(73) Proprietor: NATIONAL FOOD RESEARCH INSTITUTE MINISTRY OF AGRICULTURE, FORESTRY AND FISHERIES, Tsukuba-shi, Ibaraki-ken 305 (JP); NODA INSTITUTE FOR SCIENTIFIC RESEARCH, Noda-shi, Chiba-ken 278 (JP)
(72) Inventor: Kobayashi, Mikihiko, Tsukuba-shi, Ibaraki-ken 305 (JP); Oguma, Tetsuya, Noda Institute for Scientific Res., Noda-shi, Chiba-ken 278 (JP)
(74) Representative: Hansen, Bernd, Dr. Dipl.-Chem.

(56) References cited:
- EP-A- 0 608 636
- FR-A- 2 231 323
- FR-A- 2 515 486
- US-A- 5 002 759
- US-A- 5 071 977

## Description

### FIELD OF THE INVENTION

The present invention relates to an anti-cariogenic agent and particularly to an anti-cariogenic agent which prevents the occurrence of decay by inhibiting a glucan synthetase of Streptococcus mutans.

### BACKGROUND OF THE INVENTION

Dental plaque, a cause of dental caries (decayed teeth), is a precipitate on teeth where water-soluble and insoluble glucans produced from sucrose by Streptococcus mutans (i.e. an organism causing dental caries in the oral cavity) have formed a mixture with bacteria, etc. Dental caries by acid proceeds under dental plague. For prophylaxis of dental caries, therefore, it is extremely important to prevent dental plague from occurring. US-A-5071977 describes a hexasaccharide which is able to block the intergeneric coaggregation of two bacteria strains in the oral cavity. Moreover, a wide variety of sugars that hardly function as the substrate for the glucan synthetase of Streptococcus mutans have been exploited, and it is known that such sugars are used in confectionery instead of sucrose which is likely to function as the substrate for this enzyme.

Certainly, such sugars do not form water-soluble and insoluble glucans because they hardly function as the substrate for the glucan synthetase produced by Streptococcus mutans, but the glucan synthetase acts even with such sugars where sucrose is present. Hence, there is a need for substances with strong inhibitory action.

Hence, the object of the present invention is to provide an anti-cariogenic agent which prevents the occurrence of dental caries by inhibiting the glucan synthetase of Streptococcus mutans.

The glucan synthetase is an enzyme that synthesizes dextran by elongating short-chain dextran as the primer by transferring glucose as the hydroxyl group receptor formed from sucrose to a hydroxyl group at the 3- or 6-position of the reducing terminal glucose in said primer. Hence, the effective inhibition of this enzyme to synthesize a glucan can be expected by blocking the hydroxyl group at the 6-position of the dextran primer.

The present inventors previously found 3 kinds of cycloisomaltooligosaccharides formed from dextran, and they filed applications for patents on said substances, a synthetase for said substances, and a process for producing said substances (Japanese Patent Application Nos. 349,444/92 and 153,456/93). These cycloisomaltooligosaccharides are cyclic oligosaccharides containing 7 to 9 glucose molecules bound via α-1,6 linkages and named cycloisomaltoheptaose, cycloisomaltooctaose, and cycloisomaltononaose, respectively.

The present inventors later arrived at an idea that these cyclooligosaccharides can be prospective inhibitors of the glucan synthetase because there is no free hydroxyl group available for dextran elongation at the 6-position. As a result of their eager study on the ability of these substances to inhibit dextran synthesis, the present inventors found that the synthesis of water-soluble and insoluble glucans by the glucan synthetase produced by Streptococcus mutans can be inhibited effectively by said oligosaccharides.

### SUMMARY OF THE INVENTION

The present invention relates to an anti-cariogenic agent comprising a cycloisomaltooligosaccharide as an active ingredient.

Examples of such cycloisomaltooligosaccharides include cycloisomaltoheptaose, cycloisomaltooctaose and cycloisomaltononaose, each with a cyclic structure of glucose units bound via α-1,6 linkages.

### BRIEF DESCRIPTION OF THE DRAWINGS

FIG. 1 shows IR absorption spectra of cycloisomaltoheptaose, cycloisomaltooctaose and cycloisomaltononaose.

FIG. 2 shows chromatographic profiles of cycloisomaltoheptaose, cycloisomaltooctaose and cycloisomaltononaose after glucodextranase digestion.

FIG. 3 shows chromatographic profiles of cycloisomaltoheptaose, cycloisomaltooctaose and cycloisomaltononaose by HPLC (A, an eluate from activated charcoal; B, C and D, separated cyclooligosaccharides).

FIG. 4 shows the inhibitory effect of an increasing amount of each oligosaccharide on dextran sucrase.

FIG. 5 shows the inhibitory effect of an increasing amount of cycloisomaltooctaose on glucan synthetase from Streptococcus mutans.

### DETAILED DESCRIPTION OF THE INVENTION

Hereinafter, the 3 kinds of cycloisomaltooligosaccharides used as the active ingredient in the anti-cariogenic agent of the present invention are described.

The physicochemical properties of the 3 kinds of oligosaccharides are as follows:
1. Elementary analysis gave the following data indicating that these substances are cyclic heptamer, octamer and nonamer of glucose molecules, respectively.

| cycloisomaltoheptaose | | |
|---|---|---|
| Calculated | C, 42.43 %; | H, 6.44 % (as C₄₂H₇₀O₃₅•3H₂O). |
| Found | C, 42.78 %; | H, 6.33 %. |

| cycloisomaltooctaose | | |
|---|---|---|
| Calculated | C, 42.11 %; | H, 6.48 % (as C₄₈H₈₀O₄₀·4H₂O). |
| Found | C, 42.37 %; | H, 6.24 %. |

| cycloisomaltononaose | | |
|---|---|---|
| Calculated | C, 41.86 %; | H, 6.51 % (as C₅₄H₉₀O₄₅·5H₂O). |
| Found | C, 41.53 %, | H, 6.18 %. |

2. Mass spectroscopy with Mass Spectrometer 80B (Hitachi Seisaku) gave the following molecular weights.

| | |
|---|---|
| cycloisomaltoheptaose | 1134. |
| cycloisomaltooctaose | 1296. |
| cycloisomaltononaose | 1458. |

These molecular weights agree with those estimated from their molecular formulae.
3. Because their clear melting points could not be determined with a melting point measuring instrument manufactured by Yanagimoto, their decomposition (i.e. discoloration) temperatures are shown below.

| | |
|---|---|
| cycloisomaltoheptaose | 234 to 238 °C. |
| cycloisomaltooctaose | 238 to 241 °C. |
| cycloisomaltononaose | 239 to 242 °C. |

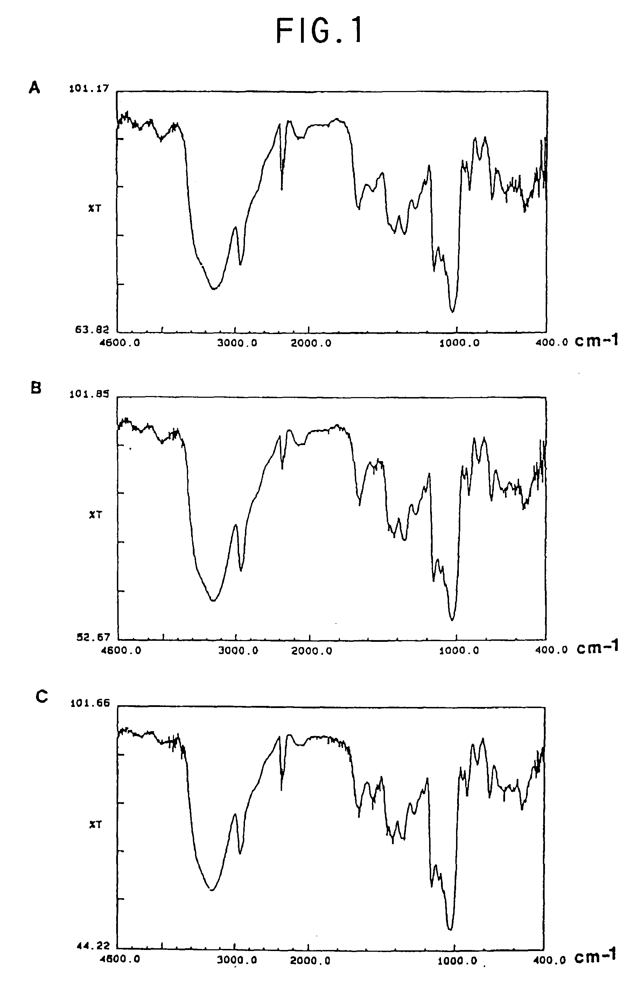

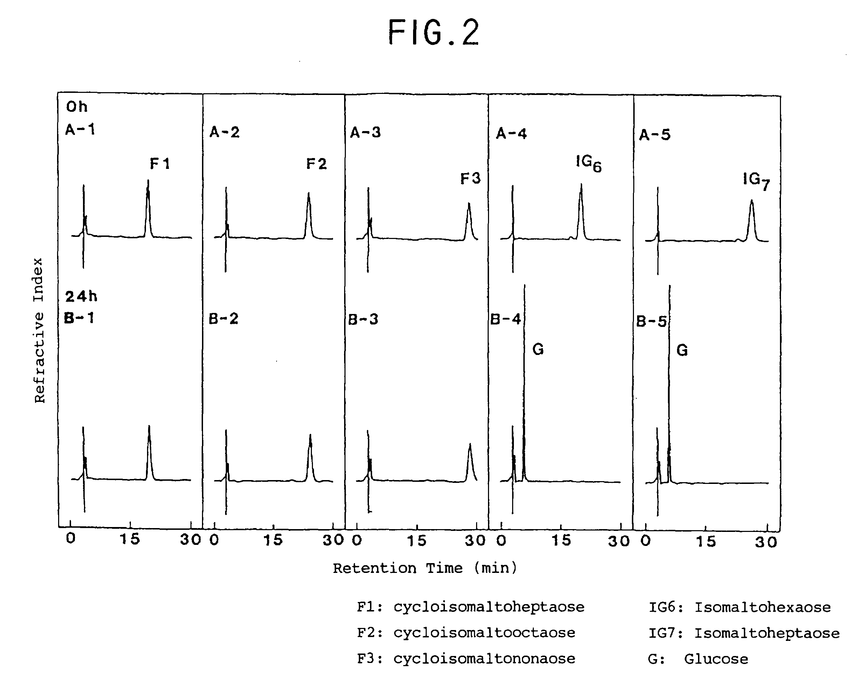

4. UV absorption spectra recorded with Spectrophotometer 775 (Hitachi Seisaku) indicated no characteristic absorption, suggesting the absence of any functional group such as amino group, carboxyl group, etc.
5. IR absorption spectra recorded with IR Spectrometer Model FT/IR-7300 (Nihon Bunko) are shown in FIG. 1 where A, B, and C correspond to cyclic heptamer, octamer and nonamer of glucose molecules, respectively. The absorption peaks at 917 ± 2 cm⁻¹ and 768±1 cm⁻¹ characteristic of α-1,6 linkage are present in the spectra, which suggests that α-1,6 linkages are present in the oligosaccharides.
6. The solubilities of the oligosaccharides in water are at least 20 mg/ml at room temperature.
7. Since no coloration occurred in the Somogyi-Nelson method, it was strongly suggested that there is no reducing terminal in any of the oligosaccharides.
8. The oligosaccharides are white substances of neutral pH.
9. Their cyclic structure was supported by the presence of only 6 signals in ¹³C-NMR spectra recorded with NMR Spectrometer Model NM-FX200 (Nippon Denshi). The analysis of isomaltoheptaose strongly suggested that the mode of binding is α-1,6 linkage.
The behaviors of the 3 kinds of oligosaccharides toward each enzyme are as follows:
10. As shown in FIG. 2, the oligosaccharides (1 % solution) were not hydrolyzed by the action of glucodextranase (exo-type dextranase) at 40 °C in 24 hours. Isomaltohexaose and isomaltoheptaose were completely hydrolyzed under the same conditions. The results suggest that they are not linear isomaltooligosaccharides.
11. The oligosaccharides (1 % solution) were decomposed into glucose and isomaltose by the action of endo-type dextranase. This result suggests that these oligosaccharides consist of glucose as the sole constitutional unit bound via α-1,6 linkage.

From the results in the above items 1 to 11, said compounds are identified as cyclooligosaccharides of 7 to 9 glucose molecules bound via α-1,6 linkages. These cycloisomaltooligosaccharides are represented by the following formula:

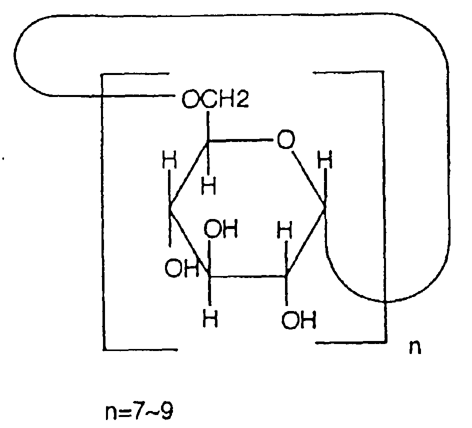

Hereinafter, a process for producing the above cycloisomaltoolig osaccharides is described.

The cycloisomaltooligosaccharides can be produced using a certain microorganism. The microorganism used may be any of the strains belonging to the genus Bacillus having the ability to convert dextran into cycloisomaltooligosaccharide, such as for example the strain T-3040.

The strain T-3040 is a wild strain obtained from the soil. Its microbial properties are as follows:

### (1) Morphological features

| | | |
|---|---|---|
| a. | Form | Bacillus |
| b. | Motility | recognizable |
| c. | Spore | present |
| | Sporangium | evagination |
| | Shape | elliptic |
| | Position | (central or subterminal) |
| d. | Gram stainability | + |

### (2) Growth states

| | | |
|---|---|---|
| a. | meat agar plate culture | smooth and no pigment occurrence |
| b. | meat agar slant culture | smooth and no pigment occurrence |

### (3) Physiological properties

| | | |
|---|---|---|
| a. Nitrate reduction | - | |
| b. Denitrification | - | |
| c. MR test | - | |
| d. VP test | - | |
| e. Indole formation | - | |
| f. Hydrogen sulfide formation | - | |
| g. Starch hydrolysis | + | |
| h. Citric acid utilization | - | |
| i. Utilization of inorganic | nitrogen sources | |
| Nitrate | - | |
| Ammonium salt | + | |
| j. Urease | - | |
| k. Oxidase | - | |
| l. Catalase | + | |
| m. Growth temperature range | 10-37°C | |
| n. Attitude toward oxygen | aerobic | |
| o. O-F test | - | (no acid production) |
| p. Attitude toward sugars | acid formation | gas formation |
| (1) L-Arabinose | - | - |
| (2) D-Xylose | - | - |
| (3) D-Glucose | + | - |
| (4) D-Mannose | - | - |
| (5) D-Fructose | - | - |
| (6) D-Galactose | + | - |
| (7) Maltose | + | - |
| (8) Sucrose | + | - |
| (9) Lactose | + | - |
| (10) Trehalose | + | - |
| (11) D-Sorbitol | - | - |
| (12) D-Mannitol | - | - |
| (13) Inositol | - | - |
| (14) Glycerin | - | - |
| (15) Starch | + | - |

Since the strain T-3040 was spore-forming Gram-positive Bacillus, it was identified as a microorganism belonging to the genus Bacillus and designated Bacillus sp. T-3040.

The strain Bacillus sp. T-3040 has been deposited as FERM BP-4132 with Fermentation Research Institute, Agency of Industrial Science and Technology, Japan.

The method of culturing this strain is the same as that used in aerobic culture of conventional organisms, and shake culture or spinner culture under aeration in liquid medium is usually used.

The medium used contains dextran etc. as the starting material of the present compounds, in addition to ingredients used for culturing conventional organisms, such as nitrogen and carbon sources, vitamins, minerals, etc.

The medium may be in any of the pH range at which the present organism can grow, preferably in the range of 6 to 8.

Shake culture or spinner culture under aeration is carried out usually at 20 to 40°C, preferably 30 °C, for 16 hours to 6 days, preferably 3 days.

From the culture thus obtained, a purified preparation of the present cycloisomaltooligosaccharides is obtained for example by the following manner.

Prior to purification, the culture is centrifuged, concentrated through membrane, etc., to give a bacteria-free culture. The purification of the oligosaccharides from the bacteria-free culture can be effected by any of the methods known in the art including cooling treatment, treatment with organic solvent, treatment with activated charcoal, or column chromatography for specifically adsorbing cyclooligosaccharides, column chromatography on activated charcoal, etc. In particular, a known method of purifying cyclodextrin can be used to obtain a preparation of high purity. If necessary, chromatographic means such as HPLC on a partition-adsorption column etc. can be further used to prepare each oligosaccharide of high purity.

The cyclooligosaccharide thus obtained may be applied as the anti-cariogenic agent to the oral cavity in itself or as toothpaste, gargle, chewing gum and troche, or as food and drink, pharmaceutical preparation, etc.

The anti-cariogenic agent of the present invention is compounded so as to reach a concentration of not less than 0.001 mM in the mouth, preferably 12.5 to 50 mM. As a troche (tablet) for example, the anti-cariogenic agent is compounded in an amount of 0.1 weight-% or more, preferably 10 to 60 weight-%.

The inhibitory action of the anti-cariogenic agent of the present invention on the glucan synthetase of Streptococcus mutans is so significant that the occurrence of dental caries is prevented very well.

### EXAMPLES

The present invention is described in more detail with reference to the following examples.

### Synthetic Example 1

3 ml liquid medium (tap water, pH 7.0) containing 1 % dextran T2000, 1 % peptone, 0.5 % NaCl and 0.1 % yeast extract was introduced into 15 ml tube and sterilized at 120 °C for 20 min. Bacillus sp. T-3040 (FERM BP-4132) from a stored slant culture was inoculated into it and cultured at 30°C for 1 day under shaking. 3 ml of the culture was transferred to a 3 L jar containing 2 L medium of the same composition sterilized under the same conditions as above and the bacteria were cultured for 2 days at 30 °C under aeration at 0.25 v.v.m. and 350 r.p.m.. Then, the culture was centrifuged at 8000 r.p.m. for 20 min. to remove the bacteria from the culture.

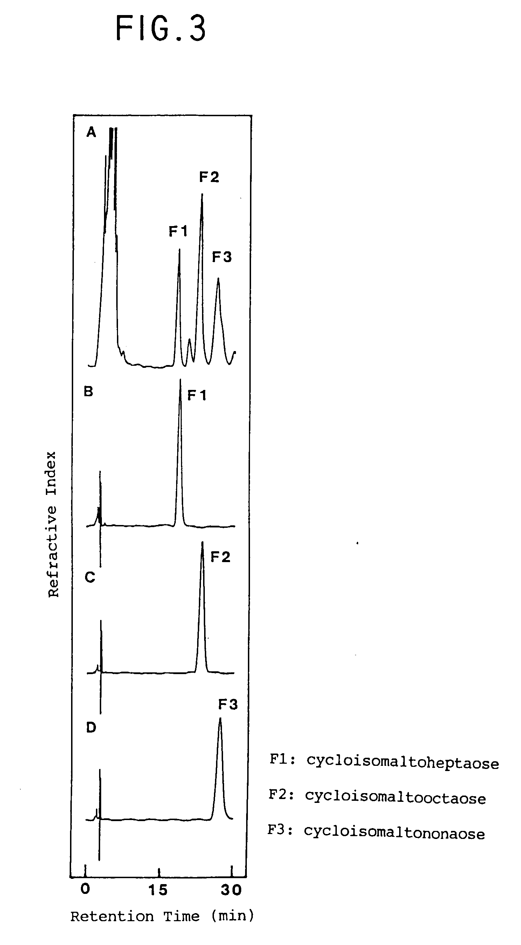

The supernatant (the bacteria-free culture) was applied to a column of activated charcoal and the adsorbed cycloisomaltooligosaccharide was eluted with a stepwise (5 %) increasing concentration of ethanol. The fraction eluted with 20 % ethanol contained the largest amount of the desired cyclooligosaccharides. This eluate was concentrated in a rotary evaporator and analyzed by HPLC on a TSK gel Amide 80 column (a partition-adsorption chromatography column, Tohso Co., Ltd.). The result is shown in FIG. 3 A. The above concentrate was subjected to HPLC on a YMC PA43 column (a preparative partition-adsorption chromatography column, Yamanura Kagaku) to separate into each cycloisomaltooligosaccharide.

A fraction of each cycloisomaltooligosaccharide was concentrated in a rotary evaporator. The concentrate was allowed to react with glucodextranase (exo-type dextranase) at 40 °C overnight to remove the linear isomaltooligosaccharides present, and the reaction solution was boiled to terminate the reaction and then centrifuged to remove denatured protein. The supernatant was subjected again to HPLC on the YMC PA43 column to give each cycloisomaltooligosaccharide. Each fraction was concentrated in a rotary evaporator and analyzed for the purity of each oligosaccharide by HPLC on a TSK gel Amide 80 column (a partition-adsorption chromatography column, Tohso Co., Ltd.). The result is shown in FIG. 3 B to D. The purity of each oligosaccharide was not less than 98 %. The respective oligosaccharide fractions were lyophilized to give about 60 mg cycloisomaltoheptaose, about 200 mg cycloisomaltooctaose and about 100 mg cycloisomaltononaose, respectively.

### Synthetic Example 2

15 ml test tube was charged with 3 ml brain heart infusion medium (BHI medium) containing 1 % Tween and sealed with a silicone stopper, and the medium was sterilized at 120°C for 10 min with humidity. 0.2 ml of a suspension of lyophilized Streptococcus mutans IFO 13955 in sterilized physiological saline was inoculated into the medium, followed by 24 hours of stationary culture at 37°C.

1 ml of the culture was transferred to 500 ml flask containing 100 ml BHI medium sterilized under the same conditions as above. After 24 hours of stationary culture at 37 °C, 10 ml of the culture was transferred to 1200 ml media bottle containing 1000 ml of the same medium sterilized under the same conditions as above. After 24 hours of stationary culture at 37 °C, the culture was centrifuged to remove the bacteria, and the supernatant was concentrated by ultrafiltration (membrane filter P-10 produced by Amicon) into a volume of about 1/20 to give a fraction of glucan synthetase from Streptococcus mutans.

### Example 1

5 to 25 µl of 50 mM each aqueous solution of the cycloisomaltoheptaose, cycloisomaltooctaose and cycloisomaltononaose prepared in Synthetic Example 2 was mixed with 25 µl purified dextran sucrase prepared according to the method described in Biochem. Biophys. Acta, 614, 46-62 (1980), and the mixture was subjected to plain incubation at 30°C for about 20 min. Then, 50 µl sucrose was added thereto as the substrate to synthesize dextran. The inhibitory effect of each cycloisomaltooligosaccharide on the dextran sucrase was determined by quantifying the resulting dextran and the reducing power occurring upon sucrose hydrolysis.

The measurement of reducing power was conducted according to the Somogyi-Nelson method. To measure the resulting dextran, it was decomposed with dextranase and quantified enzymatically with glucose oxidase.

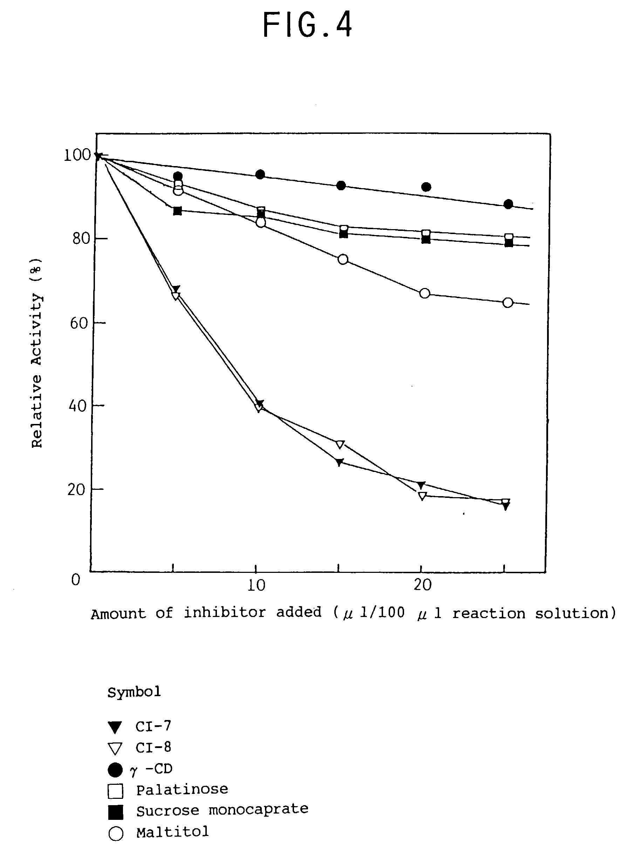

Relative activity was calculated assuming that the enzyme activity in the absence of cycloisomaltooligosaccharide is 100 %. FIG. 4 shows the inhibitory effect of an increasing amount of each cycloisomaltooligosaccharide on dextran sucrase.

### Comparative Example 1

The inhibitory effects of γ-CD (Nippon Shokuhin Kako), palatinose (Wako Pure Chemical Industries Ltd.), maltitol (Sanwa Denpun Kogyo) and sucrose monocaprate (Mitsubishi Kasei Shokuhin) on dextran sucrase were determined respectively in the same manner as in Example 1. The results are shown in FIG. 4.

### Example 2

25 µl of 5 mM each aqueous solution of the cycloisomaltoheptaose, cycloisomaltooctaose and cycloisomaltononaose prepared in Synthetic Example 1 was added to 25µl of the glucan synthetase fraction obtained from Streptococcus mutans in Synthetic Example 2, and the mixture was subjected to preincubation at 30 °C for about 20 min. Then, 50 µl of 10 % sucrose was added thereto as the substrate to examine the inhibitory effect of each cycloisomaltooligosaccharide on the glucan synthetase.

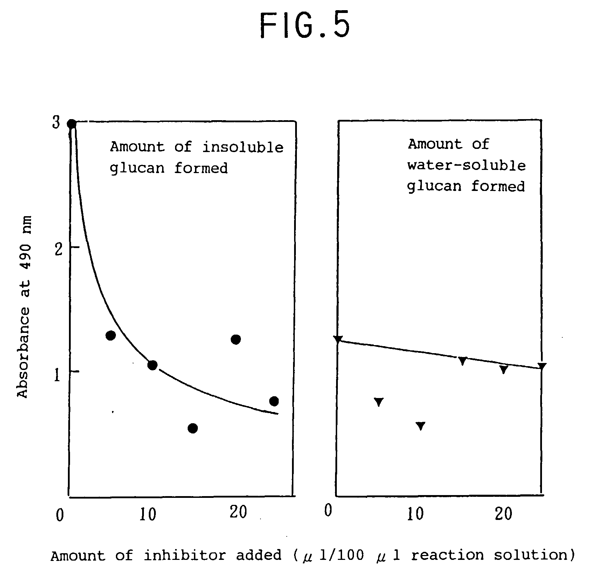

To determine the water-soluble glucan-synthesizing activity of the glucan synthetase, the insoluble glucan present was removed by centrifugation and the water-soluble glucan was precipitated with ethanol, recovered, and determined by quantifying its sugar content in the phenol-sulfuric acid method. To determine the insoluble glucan-synthesizing activity, the above insoluble glucan obtained by centrifugation was determined by quantifying its sugar content in the phenol-sulfuric acid method. FIG. 5 shows the inhibitory effect of an increasing amount of cycloisomaltooctaose on glucan synthetase.

### Example 3

### Example of pharmaceutical preparation

The cycloisomaltoheptaose, cycloisomaltooctaose and cycloisomaltononaose produced in Synthetic Example 1 were employed to prepare tablets of the following composition.

| Tablet | (weight-%) |
|---|---|
| cycloisomaltoheptaose | 15.0 |
| cycloisomaltooctaose | 25.0 |
| cycloisomaltononaose | 10.0 |
| starch | 49.4 |
| magnesium stearate | 0.6 |
| | 100 |

The cycloisomaltoheptaose, cycloisomaltooctaose and cycloisomaltononaose were mixed, followed by addition of starch (10 % starch paste), and the mixture was then formed into granules. Then, these were passed through No. 60 mesh (B.S.) screen, dried and immediately screened through No. 16 mesh (B.S.) screen. The granules thus obtained were mixed with magnesium stearate and formed by a tabulating machine into tablets of 1 cm in diameter weighing 500 mg per tablet.

### Example 4

The cycloisomaltoheptaose, cycloisomaltooctaose and cycloisomaltononaose produced in Synthetic Example 1 were employed to prepare tooth paste of the following composition.

| Toothpaste | (weight-%) |
|---|---|
| silicic anhydride | 25.0 |
| sodium carboxymethyl cellulose | 1.0 |
| sodium lauryl sulfate | 2.0 |
| glycerin | 15.0 |
| saccharin | 0.1 |
| sodium hexametaphosphate | 3.0 |
| cycloisomaltoheptaose | 1.5 |
| cycloisomaltooctaose | 5.0 |
| cycloisomaltononaose | 1.0 |
| water | remainder |
| | 100 |

A mixture of the above ingredients was adjusted to pH 7.0 with 10 % NaOH to give toothpaste.

## Claims

1. An anti-cariogenic agent comprising a cycloisomaltooligosaccharide as an active ingredient.

2. An anti-cariogenic agent according to claim 1, wherein said cycloisomaltooligosaccharide is at least one member selected from the group consisting of cycloisomaltoheptaose, cycloisomaltooctaose and cycloisomaltononaose.

3. The use of an agent according to claim 1 or 2 for the preparation of a medicament for prevention of caries in teeth.

4. The use of an agent according to claim 1 or 2 for the preparation of a medicament against dental plaque.

## Patentansprüche

1. Antikariöses Mittel, das ein Cycloisomaltooligosaccharid als wirksamen Bestandteil umfaßt.

2. Antikariöses Mittel nach Anspruch 1, dadurch gekennzeichnet, daß das Cycloisomaltooligosaccharid mindestens eine Verbindung ist ausgewählt aus der Gruppe bestehend aus Cycloisomaltoheptaose, Cycloisomaltooctaose und Cycloisomaltononaose.

3. Verwendung eines Mittels nach Anspruch 1 oder 2 zur Herstellung eines Arzneimittels zur Verhinderung von Zahnkaries.

4. Verwendung eines Mittels nach Anspruch 1 oder 2 zur Herstellung eines Arzneimittels gegen Zahnbelag.

## Revendications

1. Agent contre la formation des caries dentaires contenant en tant que principe actif un cycloisomaltooligosaccharide.

2. Agent contre la formation des caries dentaires selon la revendication 1, dans lequel ledit cycloisomaltooligosaccharide est au moins un élément choisi dans le groupe constitué par le cycloisomaltoheptaose, le cycloisomaltooctaose et le cycloisomaltononaose.

3. Utilisation d'un agent selon la revendication 1 ou 2 pour la préparation d'un médicament pour la prévention des caries dentaires.

4. Utilisation d'un agent selon la revendication 1 ou 2 pour la préparation d'un médicament contre la plaque dentaire.
